(19) Europäisches Patentamt
European Patent Office
Office européen des brevets

(11) Veröffentlichungsnummer : **0 409 009 B1**

(12) # EUROPÄISCHE PATENTSCHRIFT

(45) Veröffentlichungstag der Patentschrift :
**10.11.93 Patentblatt 93/45**

(51) Int. Cl.[5] : **C07C 323/34,** C07C 319/02

(21) Anmeldenummer : **90112890.0**

(22) Anmeldetag : **06.07.90**

(54) **Verfahren zur Herstellung von 2- und 4-Amino-thiophenolen.**

(30) Priorität : **19.07.89 DE 3923894**

(43) Veröffentlichungstag der Anmeldung :
**23.01.91 Patentblatt 91/04**

(45) Bekanntmachung des Hinweises auf die Patenterteilung :
**10.11.93 Patentblatt 93/45**

(84) Benannte Vertragsstaaten :
**CH DE FR GB IT LI**

(56) Entgegenhaltungen :
**EP-A- 0 251 552**
**DE-A- 2 706 104**
**DE-B- 2 127 898**
**HOUBEN-WEYL, Methoden der organ. Chemie, Georg Thieme Verlag Stuttgart-New York, 1985 Band E11, organ. Schwefel- Verbindungen Seite 34**

(73) Patentinhaber : **CASSELLA Aktiengesellschaft**
**Hanauer Landstrasse 526**
**D-60386 Frankfurt (DE)**

(72) Erfinder : **Bauer, Wolfgang, Dr.**
**Masurenstrasse 6**
**D-6457 Maintal 3 (DE)**
Erfinder : **Steckelberg, Willi, Dr.**
**Goldgrabenstrasse 24**
**D-6238 Hofheim (DE)**

(74) Vertreter : **Urbach, Hans-Georg, Dr. et al**
**Hanauer Landstrasse 526**
**D-60386 Frankfurt (DE)**

**EP 0 409 009 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues, mit hoher Ausbeute verlaufendes Verfahren zur Herstellung von 2- und 4-Amino-thiophenolen.

Es sind bereits verschiedene Verfahren bekannt, 2- und 4-Amino-thiophenole durch Umsetzung von 2- oder 4-Chlornitrobenzolen mit sulfidischen Reduktionsmitteln herzustellen.

Beispielsweise erfolgt nach DE 2127898 die Isolierung der Aminothiophenole aus dem stark alkalischen wäßrigen Reaktionsmedium durch Neutralisation mit einer Mineralsäure in Gegenwart von 1,5 bis 2,5 mol eines wasserlöslichen Sulfits oder 0,75 bis 1,25 mol eines wasserlöslichen Disulfits pro mol eingesetztem Chlornitrobenzol bei 0-30°C und pH-Werten von 5-7. Dabei werden Ausbeuten von 60% d.Theorie an 4-Aminothiophenol, bezogen auf 4-Chlornitrobenzol, und 71% d. Theorie an 2-Aminothiophenol, bezogen auf 2-Chlornitrobenzol, erzielt, jeweils nach Destillation der zunächst erhaltenen, stark verunreinigten Rohprodukte.

Durch den Einsatz hoher Mengen an wasserlöslichen Sulfiten bzw. Disulfiten als Reduktionsmittel vor der Isolierung der Aminothiophenole und durch die relativ geringe Ausbeute ist nach diesem Verfahren eine hohe Umweltbelastung gegeben, wodurch kostenintensive Abwasseraufarbeitungsmaßnahmen erforderlich sind. Weitere bekannte Verfahren (J. Am. Chem. Soc. 71, 1747 (1949); J. Pharm. Soc. Japan 73, 725 (1953)) liefern z.B. 4-Aminothiophenol durch Umsetzung von 4-Chlor-nitrobenzol mit Natriumsulfid in einer Ausbeute von 69% d. Theorie bzw. 72% d. Theorie (Houben-Weyl, Methoden der Organischen Chemie, Band E 11, S. 34 (1985)). 2-Aminothiophenol ist ferner durch Reduktion von Bis-(2-nitrophenyl)-disulfan mit Zinkstaub oder Zinn zugänglich (z.B. Houben-Weyl, Methoden der Organischen Chemie, Band E 11, S. 48 (1985)), jedoch sind diese Methoden nachteilig durch die hohe Umweltbelastung, welche die eingesetzten Schwermetalle verursachen.

Aufgabe vorliegender Erfindung ist es somit, ein Verfahren bereitzustellen, das die genannten Nachteile nicht aufweist und mit hohen Ausbeuten abläuft.

Vorliegende Erfindung betrifft somit ein Verfahren zur Herstellung von 2- bzw. 4-Amino-thiophenolen der allgemeinen Formel I

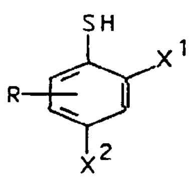

I

worin
R Wasserstoff, Methyl, Methoxy oder Chlor bedeutet und
$X^1$ und $X^2$ Amino bedeuten oder wie R definiert sind, wobei $X^1$ wie R definiert ist, wenn $X^2$ für Amino steht und $X^2$ wie R definiert ist, wenn $X^1$ für Amino steht, dadurch gekennzeichnet, daß man 2- bzw. 4-Halogennitrobenzole der allgemeinen Formel II,

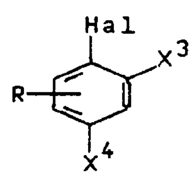

II

worin Hal Chlor oder Brom sein kann,
R wie oben definiert ist und
$X^3$ und $X^4$ Nitro bedeuten oder wie R definiert sind, wobei $X^3$ wie R definiert ist, wenn $X^4$ für Nitro steht und $X^4$ wie R definiert ist, wenn $X^3$ für Nitro steht, mit Alkalisulfiden, Alkalihydrogensulfiden oder Alkalipolysulfiden in wäßrigem alkalischen Medium umsetzt, die Reaktionsprodukte bei pH-Werten von 4 bis 8 mit einem in Wasser schwer löslichen Lösungsmittel extrahiert, nach Abtrennen der wäßrigen Phase mit wäßrigen Alkalihydroxiden nachbehandelt und die Verbindungen der allgemeinen Formel I bei pH-Werten von 4 bis 8 isoliert.

Die Umsetzung der Verbindungen der allgemeinen Formel II mit Alkalisulfiden, -hydrogensulfiden oder -polysulfiden wird bevorzugt bei Temperaturen von 40 bis 150°C, besonders bevorzugt bei 60 bis 120°C, ausgeführt, wobei gegebenenfalls auch unter Druck gearbeitet werden kann. Bevorzugte Druckbereiche sind 1 -

6 bar.

Dem wäßrigen Reaktionsmedium können beispielsweise auch mit Wasser gut oder schwer mischbare Lösungsmittel zugesetzt werden. Solche Lösungsmittel sind beispielsweise Toluol, 1,2-, 1,3- oder 1,4-Xylol, Mesizylen, Cyclohexan, Chlorbenzol, 2-Chlortoluol, 4-Chlortoluol, Tetralin, Alkohole, Glykole, Polyglykole, Glykol-mono- und -dialkylether, Polyglykol-mono- und -diglykolether, N-Methylpyrrolidon.

Bevorzugte Alkalisulfide, -hydrogensulfide und -polysulfide sind beispielsweise Natriumsulfid, Kaliumsulfid, Natriumhydrogensulfid, Kaliumhydrogensulfid, Dinatriumdisulfid and Dikaliumdisulfid.

Die Extraktion der Reaktionsprodukte mit in Wasser schwer löslichen Lösungsmitteln geschieht bevorzugt bei pH-Werten von 5 bis 7, wobei geeignete Lösungsmittel beispielsweise Hexan, Cyclohexan, Toluol, 2-, 3- oder 4-Chlor toluol, 1,2-, 1,3- oder 1,4-Xylol, Mesitylen, Tetralin, Chlorbenzol oder o-Dichlorbenzol sind.

Die sich an Extraktion und Abtrennen der wäßrigen Phase anschließende Nachbehandlung wird bevorzugt bei Temperaturen von 70 bis 180°C, besonders bevorzugt bei 100 bis 150°C, ausgeführt, wobei gegebenenfalls auch unter Druck gearbeitet werden kann. Bevorzugte Druckbereiche sind 1 - 6 bar.

Bevorzugte Alkalihydroxide sind Lithiumhydroxid, Natriumhydroxid und Kaliumhydroxid, die bevorzugt in Mengen von 0,5 bis 2 Mol, besonders bevorzugt 0,7 bis 1,5 Mol, pro Mol eingesetztes Chlornitrobenzol zugesetzt werden.

Es kann vorteilhaft sein, den wäßrigen Alkalihydroxiden bei der genannten Nachbehandlung nichtsulfidische Reduktionsmittel aus der Reihe der $\alpha$-Hydroxycarbonylverbindungen oder Natriumboranat, gegebenenfalls in Gegenwart von Phasentransferkatalysatoren zuzusetzen. Dabei werden die nichtsulfidischen Reduktionsmittel bevorzugt in einer Menge bis zu 0,3 Mol pro Mol eingesetztes Chlornitrobenzol eingesetzt. Ein höherer Einsatz dieser Reduktionsmittel ist möglich, jedoch zur Erzielung optimaler Ausbeuten nicht erforderlich. Als nichtsulfidische Reduktionsmittel aus der Reihe der $\alpha$-Hydroxycarbonylverbindungen kommen beispielsweise folgende $\alpha$-Hydroxyaldehyde und $\alpha$-Hydroxyketone sowie eine Reihe von Zuckern in Betracht, die zur Keto-Enol-Tautomerie befähigt sind: Glykolaldehyd, Glycerinaldehyd, Hydroxymalondialdehyd, Tetrosen wie Erythrose, Pentosen wie Ribose, Lyxose, Arabinose und Xylose, Hexosen wie Fructose, Glucose, Galaktose, Mannose und Allose, Hydroxyaceton, Dihydroxyaceton, Propionylcarbinol, Methylacetylcarbinol, Glucuronsäure und Ascorbinsäure.

Phasentransferkatalysatoren, die den wäßrigen Alkalihydroxiden und nichtsulfidischen Reduktionsmitteln zugesetzt werden können, kommen beispielsweise aus der Reihe der quaternären Ammonium- bzw. Phosphoniumverbindungen sowie der Kronenether, Polyethylenglykoldialkylether sowie der Kryptate (s. z.B. Ch.M. Starks u. Ch. Liotta, Phase Transfer Catalysis, Academic Press, 1978; E.V. Dehmlov, Phase Transfer Reactions, Verlag Chemie, 1980; W.P. Weber u. G.W. Gokel, Phase Transfer Catalysis in Organic Synthesis, Springer Verlag, 1977).

Die Isolierung der Verbindungen der allgemeinen Formel I geschieht bevorzugt bei pH-Werten von 5 bis 7.

Bevorzugte Ausgangsprodukte der allgemeinen Formel II sind beispielsweise 2-Chlor-nitrobenzol, 2-Brom-nitrobenzol, 4-Chlor-nitrobenzol, 4-Brom-nitrobenzol, 2,5-Dichlornitrobenzol, 3,4-Dichlor-nitrobenzol, 4-Chlor-3-nitrotoluol und 4-Chlor-3-nitroanisol.

Diese Verbindungen sind bekannt, nach allgemein bekannten Methoden herstellbar und/oder käuflich.

Das erfindungsgemäße Verfahren liefert die Verbindungen der allgemeinen Formel I in überraschend hohen Ausbeuten von 85 bis 95% d.Theorie, bezogen auf eingesetztes Chlornitrobenzol. Dabei ist die Reinheit so hoch, daß in den meisten Fällen auf eine Reinigung durch Destillation oder Kristallisation verzichtet werden kann. Das erfindungsgemäße Verfahren ist somit dem bekannten Verfahren des Standes der Technik in wirtschaftlicher, aber auch in ökologischer Hinscht, erheblich überlegen und stellt einen wesentlichen Fortschritt dar.

Die nach dem erfindungsgemäßen Verfahren hergestellten Verbindungen der allgemeinen Formel I sind wertvolle Ausgangsverbindungen zur Herstellung von Farbstoffen, Arzneimitteln, Pflanzenschutzmitteln sowie beispielsweise von aromatischen Polythioetheramiden und Epoxyharzen.

## Beispiel 1

157,6 g (1 mol) 4-Chlornitrobenzol werden in eine Mischung von 200 ml Wasser und 2,5 g Emulsogen® *) EL eingetragen und unter gutem Rühren auf 70°C geheizt. Dann wird im Verlauf von 4 Stunden eine vorher aus 221 g Natrium-sulfid, 54,4 g Schwefel und 125 ml 10 N Natronlauge in 500 ml Wasser bereitete Dinatriumdisulfid-Lösung zugegeben. Nach der Zugabe rührt man 2 Stunden bei 100°C nach und entfernt geringe Mengen an 4-Chloranilin durch Wasserdampfdestillation. Der gelborangen Lösung werden zunächst 5 g Ak-

*) (Emulsogen ist ein eingetragenes Warenzeichen der Hoechst AG, Frankfurt am Main)

tivkohle zugesetzt. Dann führt man eine Klarfiltration durch, setzt bei 20-25°C 350 ml Toluol zu und stellt durch Zugabe von 164 g 30%iger Salzsäure einen pH-Wert von 6 ein. Anschließend trennt man die gelborange Toluollösung von der wäßrigen Phase ab. Die Toluollösung wird in einen 1,3 l-VA-Autoklaven überführt und mit 270 ml Wasser sowie 140 ml 10 N Natronlauge versetzt. Anschließend heizt man unter gutem Rühren auf 130°C und rührt 4 Stunden bei 130°C nach (Druck: 3,0 bar). Das erhaltene Reaktionsgemisch wird nach Abkühlen auf 20-25°C mit 143 g 30%iger Salzsäure auf pH 6 gestellt. Man führt eine Phasentrennung durch und entfernt Toluol aus der organischen Toluolphase durch Destillation.

| | |
|---|---|
| Ausbeute: | 109,8 g |
| Schmelzpunkt: | 43-45°C |
| %-Gehalt: | 98%ig (GC) |
| Ausbeute in % d. Theorie: | 86% |

Beispiel 2

Die Herstellung der Toluol-Lösung des Primärreduktionsproduktes aus 157,6 g 4-Chlor-nitrobenzol erfolgt nach den Angaben des Beispiels 1.

Der Toluollösung werden 70 ml 10 N Natronlauge, 100 ml Wasser und 1 g Dimethyl-didecyl-ammoniumchlorid als Phasentransferkatalysator zugesetzt.

Anschließend dosiert man unter gutem Rühren bei 50-55°C eine Lösung von 7,1 g Natriumboranat in 30 ml 10 N Natronlauge und 45 ml Wasser zu. Man rührt 4 Stunden bei 50-55°C nach, kühlt dann auf 20-25°C und versetzt mit 152 g 30%iger Salzsäure bis zu einem pH-Wert von 6.

Nach Phasentrennung wird aus dem Toluol-Extrakt das Lösungsmittel durch Destillation entfernt.

| | |
|---|---|
| Ausbeute: | 109,9 g |
| Schmelzpunkt: | 44-46°C |
| %-Gehalt: | 99%ig (GC) |
| Ausbeute in % d. Theorie: | 87% |

Beispiel 3

Die Herstellung des Primär-Reduktionsproduktes aus 157,6 g 4 Chlornitrobenzol erfolgt nach den Angaben des Beispiels 1, wobei die Extraktion mit 350 ml Xylol statt 350 ml Toluol erfolgt.

Die von der wäßrigen Phase abgetrennte Xylollösung wird in einen 1,3 l VA-Autoklaven übergeführt und mit 270 ml Wasser, 140 ml 10 N Natronlauge und 31,2 g Glucose versetzt. Anschließend heizt man unter gutem Rühren auf 130°C und rührt 4 Stunden bei 130°C nach (Druck: 3,0 bar). Nach dem Abkühlen auf 20-25°C wird das Reaktionsgemisch mit 139,2 g 30%iger Salzsäure auf pH 6 gestellt.

Aus der abgetrennten Xylolphase wird anschließend das Lösungsmittel durch Destillation entfernt.

| | |
|---|---|
| Ausbeute: | 118,7 g |
| Schmelzpunkt: | 43-45°C |
| %-Gehalt: | 98%ig (GC) |
| Ausbeute in % d. Theorie: | 93% |

Beispiel 4

Man verfährt nach den Angaben des Beispiels 1, setzt jedoch statt 157,6 g 4-Chlornitrobenzol 157,6 g 2-Chlornitrobenzol ein.

Die Toluollösung des Primär-Reduktinsproduktes wird analog Beispiel 1 in einem 1,3 l VA-Autoklaven mit 140 ml 10 N Natronlauge und 270 ml Wasser bei 130° (4 Stunden) unter Druck (3 bar) nachbehandelt.

Nach Neutralisation des Reaktionsgemisches mit 30%iger Salzsäure bis zu einem pH-Wert von 5,5 und Abtrennen der Toluolphase gewinnt man 2-Aminothiophenol nach Abdestillieren des Lösungsmittels als gelbes Öl.

| | |
|---|---|
| Ausbeute: | 109,7 g |
| %-Gehalt: | 96%ig (GC) |
| Ausbeute in % d. Theorie: | 84% |

Der folgenden Tabelle sind weitere Beispiele zur Herstellung von Verbindungen der allgemeinen Formel I nach dem Verfahren des Beispiels 1 zu entnehmen, wobei in

| | |
|---|---|
| Spalte 2 | das eingesetzte Chlornitrobenzol, in |
| Spalte 3 | die Menge an Alkalisulfid, Alkalihydrogensulfid oder Alkalipolysulfid pro mol Chlornitrobenzol, in |
| Spalte 4 | die Menge Alkalihydroxid bei der Nachbehandlung in mol pro mol Chlornitrobenzol, in |

Spalte 5 das eingesetzte Reduktionsmittel in mol pro mol Chlornitrobenzol, in
Spalte 6 der eingesetzte Phasentransferkatalysator in g pro mol Chlornitrobenzol, in
Spalte 7 die Reaktionstemperatur und Zeit bei der Alkalinachbehandlung, in
Spalte 8 das Produkt und in
Spalte 9 die Ausbeute in % d.Theorie, bezogen auf eingesetztes Chlornitrobenzol
angegeben ist.

| Beispiel | Ausgangsprodukt | Primär-Reduktion: mol Alkalisulfid / mol Chlornitrobenzol | Alkalinachbehandlung: mol Alkalihydroxid / mol Chlornitrobenzol | Alkalinachbehandlung: mol Reduktionsmittel / mol Chlornitrobenzol | Alkalinachbehandlung: g Phasentransferkatalysator / mol Chlornitrobenzol | Alkalinachbehandlung: Temp./Zeit (°C) (h) | Produkt Fp. bzw. Temp. | % d.Th. |
|---|---|---|---|---|---|---|---|---|
| 5 | 1-Chlor-4-nitrobenzol | 3,1 NaHS | 1,4 KOH | - | - | 140 / 3 | 4-Aminothiophenol Fp.: 42-45° | 86 |
| 6 | 1-Chlor-4-nitrobenzol | 2,5 $Na_2S$ | 1,4 KOH | - | - | 130 / 3 | 4-Aminothiophenol Fp.: 41-43°C | 85 |
| 7 | 1-Chlor-4-nitrobenzol | 1,7 $Na_2S_2$ | 1,4 LiOH | 0,2 Hydroxyaceton | - | 130 / 2 | 4-Aminothiophenol Fp.: 43-45° | 89 |
| 8 | 1-Chlor-4-nitrobenzol | 1,7 $Na_2S_2$ | 1,4 NaOH | 0,15 Fructose | - | 130 / 4 | 4-Aminothiophenol Fp: 43-45° | 88 |
| 9 | 1-Chlor-4-nitrobenzol | 1,7 $Na_2S_2$ | 1,4 NaOH | 0,15 Ascorbinsäure | - | 130 / 4 | 4-Aminothiophenol Fp.: 43-45°C | 92 |
| 10 | 1-Chlor-4-nitrobenzol | 1,7 $Na_2S_2$ | 0,9 NaOH | 0,2 $NaBH_4$ | 2g Tributylhexadecylphosphoniumbromid | 60 / 4 | 4-Aminothiophenol Fp.: 43-46°C | 86 |
| 11 | 1-Chlor-4-nitrobenzol | 1,7 $Na_2S_2$ | 0,9 NaOH | 0,2 $NaBH_4$ | 1,5g Tris-(methoxyethoxyethyl)amin | 55 / 4 | 4-Aminothiophenol Fp.: 43-45°C | 87 |

| | | Primär-Reduktion | Alkalinachbehandlung | | | | | |
|---|---|---|---|---|---|---|---|---|
| Beispiel | Ausgangsprodukt | mol Alkalisulfid / mol Chlornitrobenzol | mol Alkalihydroxid / mol Chlornitrobenzol | mol Reduktionsmittel / mol Chlornitrobenzol | g Phasentransferkatalysator / mol Chlornitrobenzol | Temp./Zeit (°C) (h) | Produkt Fp. bzw. Temp. | % d.Th. |
| 12 | 1-Chlor-4-nitrobenzol | 1,7 $Na_2S_2$ | 0,8 NaOH | 0,2 $NaBH_4$ | 1,5g Tetraethylenglykoldimethylether | 55 / 4 | 4-Aminothiophenol Fp.: 42-45°C | 87 |
| 13 | 2-Chlor-nitrobenzol | 1,7 $Na_2S_2$ | 1,4 NaOH | 0,2 Glucose | - | 130 / 4 | 2-Aminothiophenol Kp.: 110-113°C/ 10 mbar | 89 |
| 14 | 2-Chlor-nitrobenzol | 3,1 NaHS | 1,4 NaOH | 0,15 Ascorbinsäure | - | 130 / 4 | 2-Aminothiophenol Kp.: 110-113°C/ 10 mbar | 91 |
| 15 | 2,5-Dichlor-nitrobenzol | 1,7 $Na_2S_2$ | 1,4 NaOH | 0,2 Glucose | - | 130 / 4 | 4-Chlor-2-aminothiophenol Fp.: 37-38°C | 82 |
| 16 | 4-Chlor-3-nitroanisol | 1,7 $Na_2S_2$ | 1,4 NaOH | 0,3 Glucose | - | 120 / 4 | 2-Amino-4-methoxythiophenol (Hydrochlorid, Fp.: 180-182°C) | 85 |
| 17 | 4-Chlor-3-nitrotoluol | 1,7 $Na_2S_2$ | 1,3 NaOH | 0,15 Glucose | - | 130 / 4 | 2-Amino-4-methylthiophenol (Hydrochlorid, Fp.: 186-189°C) | 87 |

**Patentansprüche**

1. Verfahren zur Herstellung von 2- bzw. 4-Amino-thiophenolen der allgemeinen Formel I

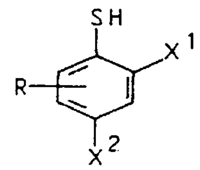

I

worin
R Wasserstoff, Methyl, Methoxy oder Chlor bedeutet und
$X^1$ und $X^2$ Amino bedeuten oder wie R definiert sind, wobei $X^1$ wie R definiert ist, wenn $X^2$ für Amino steht und $X^2$ wie R definiert ist, wenn $X^1$ für Amino steht, dadurch gekennzeichnet, daß man 2- bzw. 4-Halogennitrobenzole der allgemeinen Formel II,

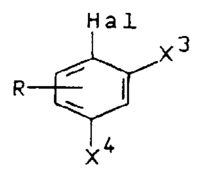

II

worin Hal Chlor oder Brom sein kann,
R wie oben definiert ist und
$X^3$ und $X^4$ Nitro bedeuten oder wie R definiert sind, wobei $X^3$ wie R definiert ist, wenn $X^4$ für Nitro steht und $X^4$ wie R definiert ist, wenn $X^3$ für Nitro steht, mit Alkalisulfiden, Alkalihydrogensulfiden oder Alkalipolysulfiden in wäßrigem alkalischen Medium umsetzt, die Reaktionsprodukte bei pH-Werten von 4 bis 8 mit einem in Wasser schwer löslichen Lösungsmittel extrahiert, nach Abtrennen der wäßrigen Phase mit wäßrigen Alkalihydroxiden nachbehandelt und die Verbindungen der allgemeinen Formel I bei pH-Werten von 4 bis 8 isoliert.

2. Verfahren nach Anspruch 1, dadurch gekennzeichnet, daß die Umsetzung der Verbindungen der allgemeinen Formel II bei Temperaturen von 40 bis 150°C, besonders bevorzugt bei 60 bis 120°C, ausgeführt wird, wobei gegebenenfalls auch unter Druck gearbeitet wird.

3. Verfahren nach Anspruch 1 und/oder 2, dadurch gekennzeichnet, daß als Alkalisulfide, -hydrogensulfide und -polysulfide Natriumsufid, Kaliumsulfid, Natriumhydrogensulfid, Kaliumhydrogensulfid, Dinatriumdisulfid oder Dikaliumdisulfid eingesetzt werden.

4. Verfahren nach einem oder mehreren der Ansprüche 1 bis 3, dadurch gekennzeichnet, daß die Extraktion mit Hexan, Cyclohexan, Toluol, 2-, 3- oder 4-Chlortoluol, 1,2-, 1,3- oder 1,4-Xylol, Mesitylen, Tetralin, Chlorbenzol oder o-Dichlorbenzol ausgeführt wird.

5. Verfahren nach einem oder mehreren der Ansprüche 1 bis 4, dadurch gekennzeichnet, daß die Extraktion bei pH-Werten von 5 bis 7 ausgeführt wird.

6. Verfahren nach einem oder mehreren der Ansprüche 1 bis 5, dadurch gekennzeichnet, daß die Nachbehandlung bei Temperaturen von 70 bis 180°C, besonders bevorzugt bei 100 bis 150°C, ausgeführt wird, wobei gegebenenfalls auch unter Druck gearbeitet wird.

7. Verfahren nach einem oder mehreren der Ansprüche 1 bis 6, dadurch gekennzeichnet, daß die Nachbehandlung mit Lithiumhydroxid, Natriumhydroxid oder Kaliumhydroxid ausgeführt wird, wobei diese in Mengen von 0,5 bis 2 Mol, besonders bevorzugt 0,7 bis 1,5 Mol, pro Mol eingesetztes Chlornitrobenzol zugesetzt werden.

8. Verfahren nach einem oder mehreren der Ansprüche 1 bis 7, dadurch gekennzeichnet, daß man den wäßrigen Alkalihydroxiden bei der Nachbehandlung nicht-sulfidische Reduktionsmittel zusetzt.

9. Verfahren nach Anspruch 8, dadurch gekennzeichnet, daß als nicht-sulfidische Reduktionsmittel α-Hydroxycarbonylverbindungen oder Natriumboranat zugesetzt werden.

10. Verfahren nach einem oder mehreren der Ansprüche 1 bis 9, dadurch gekennzeichnet, daß man den wäßrigen Alkalihydroxiden bei der Nachbehandlung Phasentransferkatalysatoren zusetzt.

## Claims

1. Process for preparing 2- and 4-amino-thiophenols of the general formula I

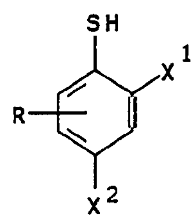

I

wherein
R denotes hydrogen, methyl, methoxy or chlorine and
$X^1$ and $X^2$ denote amino or are defined as R,
$X^1$ being defined as R if $X^2$ stands for amino and $X^2$ being defined as R if $X^1$ stands for amino,
charactericed in that 2- and 4-halogenonitrobenzenes of the formula II

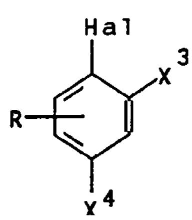

II

wherein Hal may be chlorine or bromine,
R is as defined above and
$X^3$ and $X^4$ denote nitro or is defined as R, $X^3$ being defined as R if $X^4$ stands for nitro and $X^4$ being defined as R if $X^3$ stands for nitro,
is reacted with alkali metal sulfides, alkali metal hydrogenosulfides or alkali metal polysulfides in an aqueous alkaline medium, the reaction products are extracted at pH 4 to 8 with a solvent which is sparingly soluble in water, an aftertreatment is carried out with aqueous alkali metal hydroxides after separation of the aqueous phase and the compounds of the general formula I are isolated at pH 4 to 8.

2. The process of Claim 1, characterized in that the reaction of the compounds of the general formula II is carried out at temperatures between 40 and 150°C, particularly preferably between 60 and 120°C, the reaction optionally also taking place under pressure.

3. The process of Claim 1 and/or 2, characterized in that the alkali metal sulfides, alkali metal hydrogenosulfides and alkali metal polysulfides used are sodium sulfide, potassium sulfide, sodium hydrogenosulfide, potassium hydrogenosulfide, disodium disulfide or dipotassium disulfide.

4. The process of one or several of Claims 1 to 3, characterized in that the extraction is carried out using hexane, cyclohexane, toluene, 2-, 3- or 4-chlorotoluene, 1,2-, 1,3 or 1,4-xylene, mesitylene, tetraline,

chlorobenzene or o-dichlorobenzene.

5. The process of one or several of Claims 1 to 4, characterized in that the extraction is carried out at pH 5 to 7.

6. The process of one or several of Claims 1 to 5, characterized in that the aftertreatment is carried at temperatures between 70 and 180°C, particularly preferably between 100 and 150°C, optionally also under pressure.

7. The process of one or several of Claims 1 to 6, characterized in that the aftertreatment is carried out using lithium hydroxide, sodium hydroxide or potassium hydroxide,
these being added in amounts of 0.5 to 2 mols, particularly preferably in amounts of 0.7 to 1.5, mol per mol of chloronitrobenzene employed.

8. The process of one or several of Claims 1 to 7, characterized in that in the aftertreatment non-sulfidic reducing agents are added to the aqueous alkali metal hydroxide.

9. The process of Claim 8, characterized in that the non-sulfidic reducing agents added are α-hydroxycarbonyl compounds or sodium boranate.

10. The process of one or several of Claims 1 to 9, characterized in that in the aftertreatment phase transfer catalysts are added to the aqueous alkali metal hydroxides.

## Revendications

1. Procédé de préparation de 2- et 4-aminothiophénols de formule générale I ci-dessous :

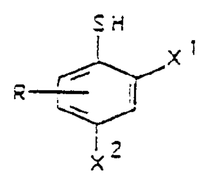

(I)

(dans laquelle
R représente l'hydrogène, un groupe méthyle ou méthoxy ou le chlore et
$X^1$ et $X^2$ sont des groupes amino ou ont les significations données pour R, $X^1$ ayant la définition de R si $X^2$ est un groupe amino et $X^2$ la définition de R si $X^1$ est un groupe amino), procédé caractérisé en ce que l'on fait réagir avec des sulfures, hydrogénosulfures ou polysulfures de métaux alcalins, dans un milieu aqueux alcalin, des 2- ou 4-halogénonitrobenzènes de formule générale II :

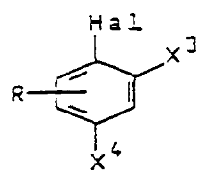

(II)

(dans laquelle Hal peut être le chlore ou le brome,
R a les mêmes significations que dans la formule I et
$X^3$ et $X^4$ désignent un groupe nitro ou ont les mêmes significations que R, $X^3$ ayant la définition de R si $X^4$ est un groupe nitro et $X^4$ la définition de R si $X^3$ est un groupe nitro), puis on extrait les produits formés à un pH de 4 à 8 avec un solvant peu soluble dans l'eau, après avoir séparé la phase aqueuse on effectue un traitement avec un hydroxyde de métal alcalin aqueux et on isole ensuite les composés de formule I à un pH de 4 à 8.

2. Procédé selon la revendication 1, caractérisé en ce que l'on effectue la réaction des composés de formule

II à des températures de 40 à 150 °C, en particulier de préférence de 60 à 120 °C, et le cas échéant en opérant sous pression.

3. Procédé selon la revendication 1 et/ou 2, caractérisé en ce que l'on prend comme sulfures, hydrogénosulfures ou polysulfures de métaux alcalins du sulfure ou de l'hydrogénosulfure de sodium ou de potassium, du disulfure disodique ou du disulfure dipotassique.

4. Procédé selon l'une ou plusieurs des revendications 1 à 3, caractérisé en ce que l'on effectue l'extraction avec de l'hexane, du cyclohexane, du toluène, du 2-, 3- ou 4-chlorotoluène, du 1,2-, 1,3- ou 1,4-xylène, du mésitylène; de la tétraline, du chlorobenzène ou de l'ortho-dichlorobenzène.

5. Procédé selon l'une ou plusieurs des revendications 1 à 4, caractérisé en ce que l'on effectue l'extraction à un pH de 5 à 7.

6. Procédé selon l'une ou plusieurs des revendications 1 à 5, caractérisé en ce que l'on effectue le traitement complémentaire à des températures de 70 à 180 °C, notamment de préférence de 100 à 150 °C, et le cas échéant en opérant sous pression.

7. Procédé selon l'une ou plusieurs des revendications 1 à 6, caractérisé en ce que l'on effectue le traitement complémentaire avec de l'hydroxyde de lithium, de sodium ou de potassium dans des proportions de 0,5 à 2 mol, notamment de préférence de 0,7 à 1,5 mol, par mol du chloronitro- benzène employé.

8. Procédé selon l'une ou plusieurs des revendications 1 à 7, caractérisé en ce que l'on ajoute aux hydroxydes de métaux alcalins aqueux, dans le traitement avec ces composés, des agents réducteurs non-sulfurés.

9. Procédé selon la revendication 8, caractérisé en ce que l'on ajoute comme réducteurs non-sulfurés des composés $\alpha$-hydroxycarbonyliques ou du boranate de sodium.

10. Procédé selon l'une ou plusieurs des revendications 1 à 9, caractérisé en ce que l'on ajoute aux hydroxydes alcalins aqueux pour le traitement complémentaire des catalyseurs de transfert de phases.